# EUROPÄISCHE PATENTANMELDUNG

(11) **EP 2 319 489 A2**
(43) Veröffentlichungstag der Anmeldung: **11.05.2011**
(21) Anmeldenummer: 10165433.3
(22) Anmeldetag: 25.04.2003
(51) Int. Cl.: A61K 8/64, A61Q 11/00

(54) **Mundpflegemittel enthaltend Ovomucin und Mannoprotein**

(30) Priorität: 25.04.2002 CH 7072002
(62) Teilanmeldung aus: 03714599.2
(71) Anmelder: Gaba International AG, 4106 Therwil (CH)
(72) Erfinder: Garbers, Christine, 4059, Basel (CH); Merck, Karin Beatrice, 5345 VX, Oss (NL); Kleter, Gijsbertus Anthonius, 6703 AT, Wageningen (NL); Vereijken, Johannes Maria, 6721 TV, Bennekom (NL); Rasing, Gerardus Ferdinandus Johannes, 3527 WB Utrecht (NL)
(74) Vertreter: Kaufmann, Michael Charles

(57) **Zusammenfassung**

Es werden Mundpflegemittel, insbesondere Speichelersatzflüssigkeit beschrieben, die ein Ovomucin enthaltendes Schleimmittel und Mannoprotein enthalten.

## Beschreibung

Die vorliegende Erfindung betrifft Mundpflegemittel. Insbesondere betrifft sie solche Mundpflegemittel in Form von Speichelersatzflüssigkeiten.

Im Hinblick auf die Feuchthaltewirkung auf die Mundschleimhäute enthalten Mundpflegemittel üblicherweise ein Feuchthaltemmittel, z.B. mehrwertige Alkohole wie Glycerin, Propylenglykol, Sorbit, Mannit, Glucosesirup; Polyethylenglykole, Polypropylenglykole; Polyvinylpyrrolidon; oder Cellulosederivate wie etwa Hydroxyethylcellulose.

Mundpflegemittel in Form von Speichelersatzflüssigkeiten werden im Fall eines ungenügenden natürlichen Speichelflusses eingesetzt. Der in der Fachsprache als "Xerostomie" bezeichnete krankhafte ungenügende Speichelfluss bewirkt, dass der Betroffene beispielsweise Mühe beim Sprechen, Kauen und Schlucken hat. Er muss während des Sprechens oft einen Schluck Wasser oder eines Getränkes zu sich nehmen, um die Feuchtigkeit der Mundhöhle wieder herzustellen, was aber nur kurz wirkt. Xerostomiker sind anfälliger für Infektionen der Mundhöhle, insbesondere auch der Speichelgänge.

Bei einer Speichelersatzflüssigkeit muss nicht nur die Feuchthaltewirkung auf die Mundschleimhäute erzielt werden, sondern es muss bevorzugt auch das richtige rheologische Verhalten der Speichelersatzflüssigkeit erzielt werden. Menschlicher Speichel ist eine thixotrope Flüssigkeit, d.h. seine Viskosität nimmt bei zunehmender Scherbeanspruchung ab. Diese Eigenschaft ist wichtig für seine Wirkung als Schmiermittel bei der Artikulation und beim Kauen. Die Thixotropie des Speichels wird durch darin enthaltene Glykoproteine (Mucine) bewirkt. Das Mucin, das im menschlichen Speichel vorkommt, weist typisch 50 bis 90 Gewichtsprozente Kohlenhydrate und etwa 3,9 mg / 100 g Sialinsäure auf und hat ein Molekulargewicht von typisch etwa 200 bis etwa 1000 kDa.

Auf dem Markt sind Speichelersatzflüssigkeiten mit unterschiedlichen Feuchthalte- und/oder Schleimmitteln erhältlich. Beispiele hierfür sind (in Klammern das darin enthaltene aktive Agens): "Glandosane" der Cell Pharm GmbH, Deutschland (Cellulose), "Xialine" der Lommerse Pharma, Holland (Xanthanharz), "Stoppers 4 Dry Mouth" der Woodridge Labs, USA (Glyzerin und Hydroxyethylcellulose), "Oralbalance" der Laclede International, Belgien (Stärke und Hydroxyethylcellulose), und "Saliva medac" der Medac Gesellschaft für klinische Spezialpräparate, Deutschland (Mucin aus Schweinemagen). Bei Speichelersatzflüssigkeiten, die Mucine tierischer Herkunft als Schleimmittel enthalten, ist die Akzeptanz der Patienten fraglich, die von der Vorstellung, ein Präparat in den Mund zu nehmen, das beispielsweise aus der Schleimhaut eines Schweinemagens stammt, abgeschreckt werden können.

Das Mucin Ovomucin ist als Stoff (besser als Stoffgemisch) seit langem bekannt (z.B. Robinson, D.S., Monsey, J.B., The Biochemical Journal 1966, 100/2, 61f., und darin zitierte Literatur). Es kommt im diversen Bestandteilen von Eiern, insbesondere dem Eiklar, vor. Typisch werden aus dem rohen Eiklar (vorgängig durch Sieben von der Hagelschnur und von den Eimembranen getrennt) zunächst durch Verdünnen in mehreren Volumina Wasser und lösliche Proteine, die lösliche Fraktion des Eiklars, Salze, und sonstige niedermolekulare Verbindungen herausgelöst. Die als Gel übrigbleibende dicke Fraktion des Eiklars wird mit weiterem Wasser und/oder mit KCl-Lösung gewaschen, wobei das Ovomucin ausfällt. Dieses Ovomucin enthält Untereinheiten, die als α-und β-Ovomucin bezeichnet werden. Das α-Ovomucin weist N-glykosidisch gebundene Kohlenhydrate (ca. 15 Gewichtsprozent) und nur wenig Sialinsäure auf. Das β-Ovomucin weist O-glykosidisch gebundene Kohlenhydrate (ca. 50 bis 60 Gewichtsprozente) und viel Sialinsäure auf. Neben den eigentlichen Ovomucin-Glycoprotein sind of auch weitere nicht-mucine Proteine, insbesondere auch Lysozym, vorhanden (dessen Abtrennung von dem eigentlichen Ovomucin ist schwierig, da es mit letzteren interagiert). Es handelt sich bei Ovomucin also nicht um eine Reinsubstanz, sondern um ein nicht genau charakterisierbares Substanzgemisch, in dem das eigentliche Ovomucin-Glycoprotein mehr oder weniger dominant vorkommt.

Das isolierte Ovomucin ist zunächst in Wasser sehr schwer oder nicht löslich. Der Grund liegt in der Oxidation von Thiolgruppen des in Ovomucin enthaltenen Cysteins unter Ausbildung von Disulfidgruppen. Dabei werden Quervernetzungen zwischen den einzelnen Mucinketten ausgebildet, die zu extrem hochmolekularen Polymeren führen. Die Löslichmachung des Ovomucins nach der Isolation wird in der Regel durch reduzierende Agentien wie Mercaptoethanol (Rückreduktion der Disulfidbrücken zu Thiolen) und wahlweise durch zusätzlichen Einsatz von thiolblockierenden Agentien wie HgCl₂, Quecksilber-bis(p-chlorbenzoat) oder Jodessigsäure bewirkt. Diese Agentien sind jedoch allesamt toxisch. Es ist auch bekannt, dass die Löslichkeit von Ovomucin mit zunehmendem Gehalt der Lösung an NaCl (bis etwa 2 M) zunimmt (Rabouille, C., Aon, M.A., Thomas, D., Archives of Biochemistry and Biophysics, 1989, 270(2), 495-503). Solche Salzgehalte sind aber in Mundpflegemitteln nicht brauchbar.

Die rheologischen Eigenschaften von Ovomucin-Lösungen, wahlweise auch in Kombination mit einem einzelnen weiteren Zusatz, ausgewählt aus Lysozym, NaCl oder CaCl₂, wurden untersucht (Hayakawa, S., Sato, Y., Agric. Biol. Chem. 1978, 42(11), 2025-2029).

Es ist bekannt, dass im Eiklar ein Komplex zwischen Ovomucin und dem nicht-mucinen Protein Lysozym vorliegt (u.a. Miller, S.M., Kato, A., Nakai, S., J. Agric. Food Chem. 1982, 30, 1127-1132).

In der WO-A-99/04804 werden Mundpflegemittel, beispielsweise Speichelersatzflüssigkeiten, beschrieben, die u.a. entovalbuminisiertes Eiklar und/oder wässeriger Extrakt von Eigelb enthalten können. Die WO-A-99/04804 betont die Bedeutung der immunologisch wirkenden Bestandteile des Eis, z.B. Lysozym und Ovotransferrin, in den Mundpflegemitteln.

Von einigen der im Eiklar vorhandenen Proteine, z.B. von Ovalbumin, Lysozym und Ovotransferrin, ist bekannt, dass sie als Allergene wirken können.

Aufgabe der vorliegenden Erfindung ist es, Mundpflegemittel mit guter Feuchthaltewirkung auf die Mundschleimhaut bereitzustellen, die günstiger sind in der Akzeptanz der Patienten als die vorbekannten Mundpflegemittel, bei denen Mucine tierischer Herkunft für Feuchthaltwirkung sorgen, und die eine verringerte Gefahr allergischer Reaktionen mit sich bringen. Insbesondere ist es Aufgabe der vorliegenden Erfindung, solche verbesserte Speichelersatzflüssigkeiten bereitzustellen, die auch bevorzugt den rheologischen Eigenschaften des menschlichen Speichels nahe kommen.

Die Aufgabe wird durch Mundpflegemittel gelöst, die umfassen:
a) ein Schleimmittel, das Ovomucin enthält und einen Sialinsäuregehalt von 1,5 bis 2,5 Gewichtsprozenten aufweist, und
b) einen Emulgator.

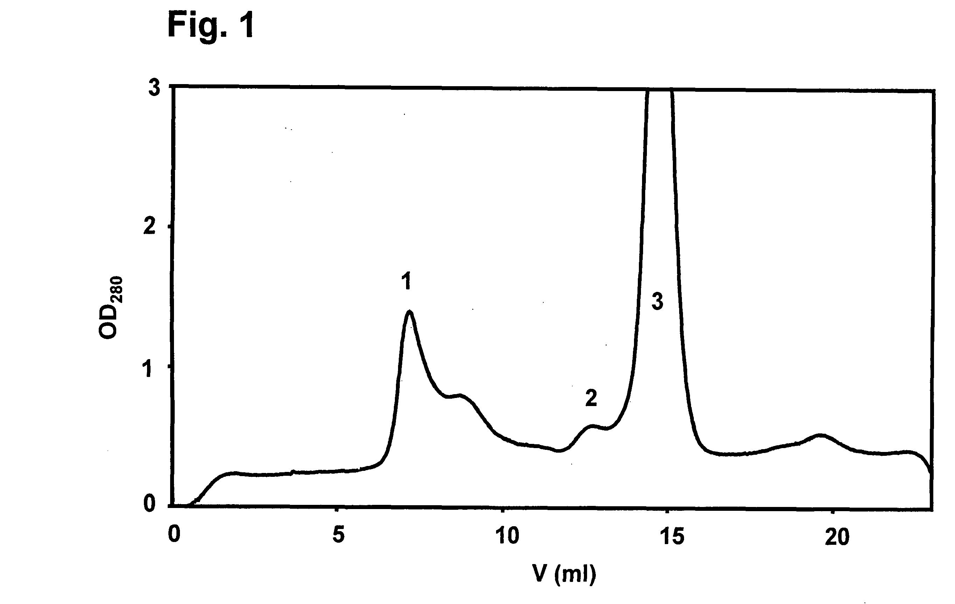

Figur 1 ist eine chromatographische Auftrennung von a) einem erfindungsgemäss verwendbaren Ovomucin enthaltenden Schleimmittel, wie in Beispiel 1 hergestellt. Die Bedingungen der Chromatographie sind gemäss Beispiel 2. X-Achse: eluiertes Volumen, in ml; Y-Achse: optische Dichte des Eluates, bei 280 nm gemessen. Identifizierte Verbindungen: 1 Ovomucin; 2 Ovalbumin (43 kDa) und Ovotransferrin (77 kDa); 3 Lysozym (14 kDa).

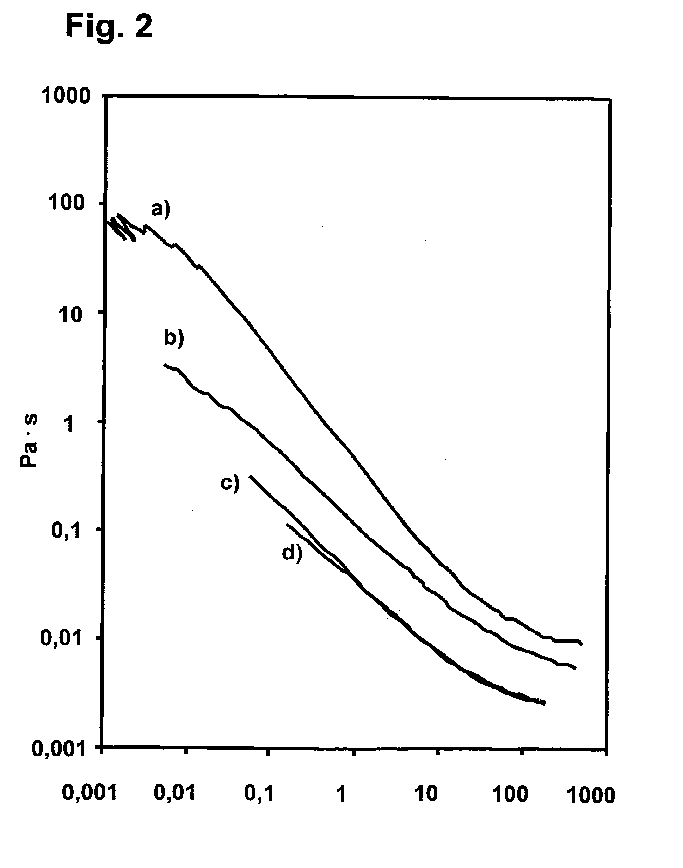

Figur 2 ist eine Darstellung des rheologischen Verhaltens verschiedener Schleimmmittel bei 37°C. X-Achse: Schergeschwindigkeit in Umdrehungen pro Sekunde; Y-Achse: gemessene Viskosität in Pascal x Sekunden. Es bedeuten: a) Getrocknete Probe eines Ovomucin enthaltenden Schleimmittels, wie in Beispiel 1 hergestellt, b) Mucin aus Schweinemägen, von Sigma bezogen, c) und d) zwei verschiedene Proben Mucin aus Schweinemägen, von ICN bezogen.

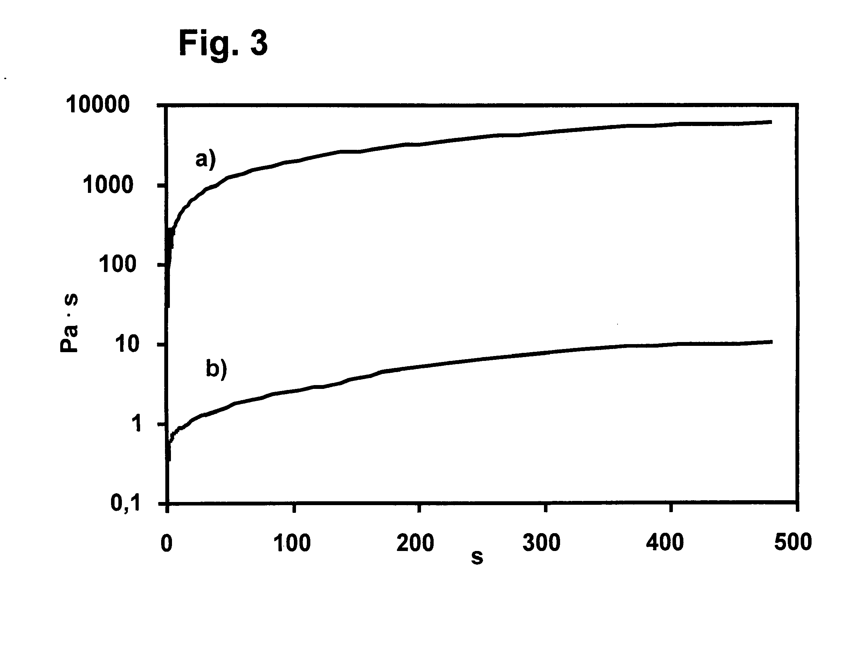

Figur 3 zeigt den zeitlichen Ablauf der Wiederherstellung der Viskosität bei 37°C nach hoher Scherbeanspruchung. X-Achse: Zeitskala in Sekunden, Y-Achse: Viskosität in Pascal x Sekunden. Es ist a) das Ovomucin enthaltende Schleimittel von Beispiel 1, und b) ein Mucin aus Schweinemagen (bezogen von Sigma).

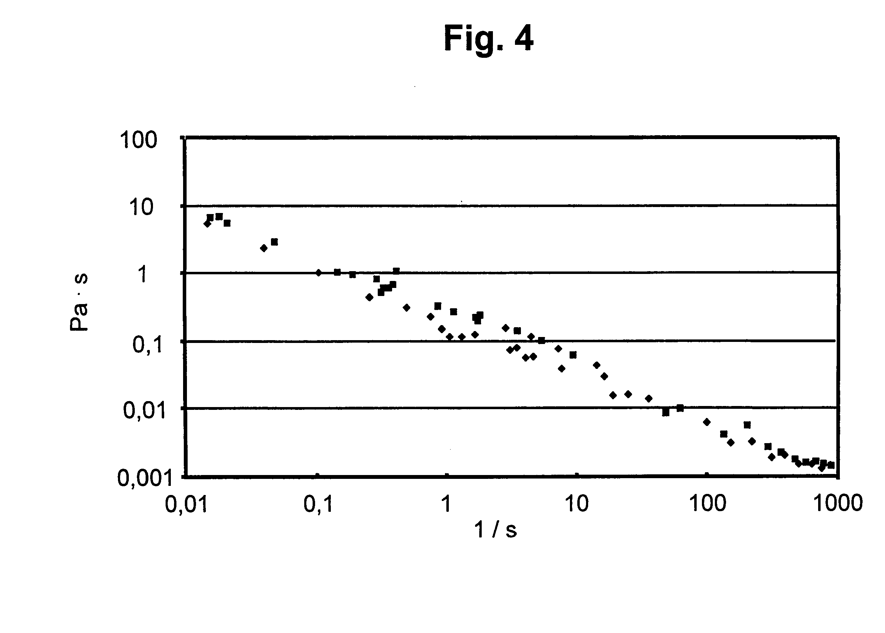

Figur 4 ist eine Darstellung des rheologischen Verhaltens bei 37°C von zwei Proben (Rhomben bzw. Quadrate) einer Speichelersatzflüssigkeit (knapp 2 Gewichtsprozent Trockenmasse Ovomucin enthaltendes Schleimmittel, bezogen auf das Gewicht der Flüssigkeit) mit 0,15 Gewichtsprozenten Cremophor ® RH 410 als Emulgator. X-Achse: Schergeschwindigkeit in Umdrehungen pro Sekunde; Y-Achse: Gemessene Viskosität in Pascal x Sekunden.

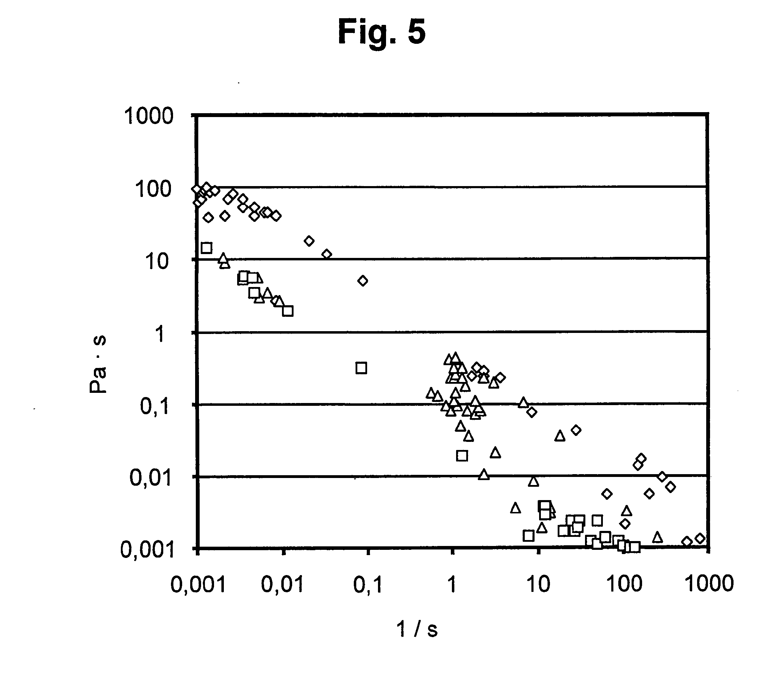

Figur 5 ist eine Darstellung des rheologischen Verhaltens bei 37°C von drei Proben (Dreiecke, Rhomben, Quadrate) einer Speichelersatzflüssigkeit (knapp 2 Gewichtsprozent Trockenmasse Ovomucin, bezogen auf das Gewicht der Flüssigkeit) mit 0,15 Gewichtsprozenten Tween 20 ®. X-Achse: Schergeschwindigkeit in Umdrehungen pro Sekunde; Y-Achse: Gemessene Viskosität in Pascal x Sekunden.

Es wurde überraschenderweise gefunden, dass durch die Verwendung eines Schleimmittels, das Ovomucin enthält oder im Wesentlichen aus diesem besteht, Mundpflegemittel erhalten werden, die die Mundschleimhaut wirksam feucht halten. Es wurde auch gefunden, dass bei Verwendung eines solchen Schleimmittels in Mundpflegemitteln in Form einer Speichelersatzflüssigkeit gleichzeitig ein brauchbares rheologisches Verhalten einer solchen erfindungsgemässen Speichelersatzflüssigkeit erhalten wird. Es wurde auch gefunden, dass die Löslichkeitsverringerung des im erfindungsgemäss einzusetzenden Schleimmittel enthaltenen Ovomucins, die durch die weitgehende Entfernung der möglicherweise allergenen Proteine Ovalbumin, Lysozym und Ovotransferrin bewirkt ist, durch Zugabe eines Emulgators kompensiert werden kann. Es wurde des Weiteren im Falle der erfindungsgemässen Speichelersatzflüssigkeiten gefunden, dass die Zugabe des Emulgators das rheologische Verhalten des im Schleimmittels enthaltenen Ovomucins nicht nachteilig beeinflusst, d.h. das thixotrope Verhalten des Ovomucins bleibt im Wesentlichen unverändert erhalten. Der Begriff "Schleimmittel" bedeutet im Rahmen der vorliegenden Anmeldung einen viskositätsverändernden Zusatz für Mundpflegemittel.

Als "Mundpflegemittel" wird in Rahmen der vorliegenden Anmeldung jedes Mundpflegemittel verstanden, in dem üblicherweise ein Schleimmittel vorhanden ist, und in dem erfindungsgemäss anstelle dieses Schleimmittels (oder zusätzlich zu diesen) ein Ovomucin enthaltendes Schleimmittel verwendet werden kann, wobei die restlichen Zusätze und/oder Wirksubstanzen nach Art und Menge in Analogie zu dem jeweiligen vorbekannten Mundpflegemittel gewählt werden können. Beispiele für erfindungsgemässe Mundpflegemittel sind Spüllösungen, Touchierlösungen und insbesondere Speichelersatzflüssigkeiten.

Erfindungsgemässe Spüllösungen sind vorzugsweise wässerige oder alkoholische, besonders bevorzugt gemischt wässerig/alkoholische Lösungen. Sie können typische eine relativ verdünnte Konzentration einer Wirksubstanz, z.B. eines üblichen desinfizierenden Mittels wie etwa Chlorhexidin oder Triclosan enthalten. Typische Zusätze für Spüllösungen, neben dem als Feuchthaltemittel eingesetzten Ovomucin und den aktiven Wirkstoffen, sind beispielsweise Süssstoffe wie Saccharin, quaternäre Ammoniumsaccharinate, Cyclamate, oder Aromastoffe wie beispielsweise Cumarin und Vanillin oder ätherische Öle, z.B. Pfefferminzöl, Spearmintöl, Anisöl, Menthol, Anethol, Citrusöl, usw., oder sonstige Aromen wie Apfel-, Eukalyptus- oder Krauseminzaroma.

Erfindungsgemässe Touchierlösungen können den Spüllösungen ähnlich sein, weisen aber, in Analogie zu den vorbekannten Touchierlösungen, eine relativ hohe Menge an aktivem Agens auf.

Erfindungsgemässe Speichelersatzflüssigkeiten können wässerige Lösungen sein, die das Ovomucin enthaltende Schleimmittel und diverse Salze zur Einstellung der Tonizität, der Osmolarität und des pH's enthalten dergestalt enthalten, dass eine dem menschlichen Speichel ähnliche chemische Zusammensetzung resultiert. Solche Zusätze sind Chloride der Alkali- oder Erdalkalimetalle, (beispielsweise NaCl oder MgCl₂) zur Einstellung der Tonizität sowie Puffersubstanzen wie etwa Phosphat- oder Carbonatpuffer, die den pH der Speichelersatzflüssigkeit auf einen pH einstellen, der zwar in der Mundhöhle physiologisch verträglich ist, aber genügend hoch ist, um der Auflösung des Zahnschmelzes entgegenzuwirken. Auch die erfindungsgemässen Speichelersatzflüssigkeiten enthalten analog solche Zusätze.

Erfindungsgemässe Speichelersatzflüssigkeiten weisen demnach bevorzugt 0,05 bis 0,15 Gewichtsprozente, besonders bevorzugt etwa 0,12 Gewichtprozente KCl; bevorzugt 0,05 bis 0,1, besonders bevorzugt etwa 0,086 Gewichtsprozente NaCl; bevorzugt 0,002 bis 0,008, besonders bevorzugt etwa 0,005 Gewichtsprozente MgCl₂; bevorzugt 0,01 bis 0,02, besonders bevorzugt etwa 0,015 Gewichtsprozente CaCl₂ auf; und sie sind bevorzugt mit Dihydrogenphosphat/Hydrogenphosphat und/oder Carbonat-/Hydrogencarbonat-Puffer auf einen pH-Wert und eine Pufferkapazität gepuffert, die dem menschlichen Speichel entspricht. Bevorzugt können die erfindungsgemässen Speichelersatzflüssigkeiten Calciumsalze in Kombination mit Phosphat dergestalt enthalten, dass der Auflösung des Zahnschmelzes ebenfalls entgegengewirkt wird.

Die erfindungsgemässen Mundpflegemittel enthalten nur diejenigen Mengen an Ovalbumin, Lysozym und Ovotransferrin, die als unvermeidbare Verunreinigung in dem aus Eiklar isolierten, Ovomucin enthaltenden Schleimmittel vorhanden sind. Bei der Herstellung der erfindungsgemässen Mundpflegemittel werden keine weiteren, aus Ovalbumin, Lysozym oder Ovotransferrin ausgewählten oder diese enthaltenden Zusätze verwendet.

Das erfindungsgemäss einzusetzende, Ovomucin enthaltende Schleimmittel ist einerseits das Präzipitat, das aus Eiklar (beispielsweise und vorzugsweise aus Eiklar von Hühnereiern) durch Verdünnen mit mehreren, typisch 2 bis 10 Volumina Wasser, Stehenlassen dieser Lösung zwecks Ausfällung bei typisch 0°C bis etwa Raumtemperatur und anschliessendem Abzentrifugieren erhalten werden kann. Die Ausfällung aus der wässerigen Lösung von Eiklar kann beispielsweise durch Einstellen des pH's der Lösung auf etwa den isoelektrischen Punkt des im Ovomucin enthaltenen Ovomucin-Glycoproteins (d.h. auf etwa 4,0 bis etwa 6,0, bevorzugt auf etwa 5,0 bis etwa 6,0) begünstigt werden. Es können auch leicht oxidierende Bedingungen (z.B. Luftsauerstoff) gewählt werden, die die Oxidation der Thiolgruppen der Ovomucin-Glycoproteine zu Disulfidbrücken (intra- und intermolekular) verknüpfen, wodurch ebenfalls eine Verringerung der Löslichkeit des Ovomucins erzielt wird. Diese beiden Varianten sind reversibel (durch Verschieben des pH's aus dem isoelektrischen Gebiet heraus bzw. durch Re-Reduktion der Disulfidbrücken mittels beispielsweise überschüssigem 2-Mercaptoethanol).

Erfindungsgemäss einsetzbare, Ovomucin enthaltende Schleimmittel können auch aus anderen Bestandteilen von Eiern, insbesondere von den Hagelschnüren und den Eidottermembranen, isoliert werden. Diese Bestandteile sind im Wesentlichen ein Ovomucin von höherem Polymerisierungsgrad als das Ovomucin aus Eiklar. Insbesondere das Ovomucin aus den Hagelschnüren und den Eidottermembranen ist hinsichtlich der chemischen Zusammensetzung, beispielsweise des Sialinsäuregehaltes, dem Ovomucin aus Eiklar sehr ähnlich. Zur Isolation eines erfindungsgemäss verwendbaren Schleimmittels aus Hagelschnüren oder Eidottermembranen können diese zur Abtrennung allfälliger Feststoffe (Reste von Eischalen), sofern erforderlich, vorgängig zentrifugiert werden und anschliessend einer Waschung mit wiederum typisch 2 bis 10 Volumina Wasser unterzogen werden. Bei der Herstellung eines erfindungsgemässen Mundpflegemittels wird vorteilhaft eine möglichst gute Dispergierung des Ovomucin enthaltenden Schleimmittels (z.B. durch Mischen unter hoher Scherwirkung, beispielsweise mit einem Ultraturrax ® Mischer) bewirkt, und vorteilhaft können allfällige unlösliche Reste anschliessend durch Zentrifugation, Sedimentation oder Filtration abgetrennt werden.

Beim Waschen mit typisch 2 bis 10 Volumina Wasser wird ein Grossteil der nicht-mucinen, möglicherweise allergenen Proteine (z.B. Ovalbumin, Ovotransferrin, Lysozym) aus dem Schleimmittel entfernt, da diese besser löslich sind als das Ovomucin. Dies äussert sich darin, dass der Gehalt an Sialinsäure (ausgedrückt in Gramm Sialinsäure pro 100 Gramm Trockenmasse, messbar durch kolorimetrischem Test mit saurem Ninhydrid-Reagens, siehe Yao, K., Ubuka, T., Masuoka, N., Kinuta, M., Ikeda, T., Anal. Biochem. 1989, 179, 332-335, diese Veröffentlichung ist durch Bezugnahme eingeschlossen) gegenüber dem Gehalt im ursprünglichen Eiklar stark ansteigt. Das so isolierte, Ovomucin enthaltende Schleimmittel enthält dann typisch etwa 1,5 bis etwa 2,5 Gramm Sialinsäure pro 100 Gramm Trockenmasse, bevorzugt etwa 1,7 bis etwa 2,0 Gramm pro 100 Gramm Trockenmasse. Wenn man, in Anlehnung an die Literatur, einen Gehalt von etwa 2 Gewichtsprozenten Sialinsäure für reines Ovomucin annimmt, kann davon ausgegangen werden, dass das so isolierte Schleimmittel im Wesentlichen, also zu mindestens etwa 80 Gewichtsprozenten der Trockenmasse, mutmasslich sogar zu mehr als 90 Gewichtsprozenten, aus Ovomucin besteht.

Gewünschtenfalls kann das Ovomucin enthaltende Schleimmittel durch weiteres Waschen mit Wasser und/oder KCl-Lösungen wie allgemein bekannt weiter gereinigt werden. In den Beispielen und Figuren wurde ein so weitergereinigtes, Ovomucin enthaltendes Schleimmittel aus Eiklar untersucht. Nach Kenntnis der Erfinder bewirkt das weitere Waschen aber keine weitere wesentliche Änderung des Sialinsäuregehaltes oder des Verhältnisses der Extinktionen bei 280 nm und bei 214 nm (dieses ist ein Mass für den Restgehalt insbesondere an Lysozym). Dies zeigt, dass bereits bei ersten Verdünnen des Eiklars mit typisch 2 bis 10 Volumina Wasser, Stehenlassen und Zentrifugieren der weitaus grösste Teil der Fremdproteine und Salze entfernt werden. Es ist also erfindungsgemäss nicht zwingend erforderlich, eine weitergehende Reinigung des Ovomucin enthaltenden Schleimmittels vorzunehmen.

Es wird vermutet, dass der im Schleimmittel vorhandene Restgehalt Lysozym sowie die Art und Menge der für ein Mundpflegemittel, insbesondere für eine Speichelersatzflüssigkeit typischen Salze die Löslichkeit des im Schleimmittel enthaltenen Ovomucins in den erfindungsgemässen Mundpflegemitteln erhöhen. Die Löslichkeit des Ovomucins kann, sofern erforderlich, auch durch nichttoxische Reduktionsmittel für Disulfidbrücken wie etwa Cystein, reduzierende Vitamin C-Derivate oder NADPH erhöht werden. Das Schleimmittel kann vor dem Einsatz in den erfindungsgemässen Mundpflegemitteln mit diesen Agentien digeriert werden oder es können in günstigen Fällen geeignete Mengen der Agentien als Zusätze direkt zugegeben werden.

Die Löslichkeit des Ovomucin enthaltenden Schleimmittels wird durch Emulgatoren, wie sie üblicherweise zur Dispergierung von schlecht wasserlöslichen Aromastoffen in Mundpflegemitteln verwendet werden (insbesondere synthetische Emulgatoren), erhöht. Beispiele für solche Emulgatoren sind anionische Emulgatoren (z.B. Alkalimetallsalze von Fettsäuren oder von Fettalkoholsulfaten, so etwa Natriumlaurylsulfat) oder zwitterionische Emulgatoren. Beispiele für letztere sind die Amide von (C₁₀-C₂₀) Fettsäuren mit N',N'-Dialkyl-N'-Carboxymethyl-(C₂-C₄)diaminoalkylen als amidbildendem Rest, wobei die beiden Aminogruppen des Diaminoalkylens endständig sind. Ein besonders bevorzugtes Beispiel hierfür ist Cocamidopropylbetain. Bevorzugt sind auch nichtionische Emulgatoren wie etwa die Poly(oxyethylen)derivate eines teilweise veresterten (C₃-C₆)Zuckeralkohols, wobei die den Zuckeralkohol veresternden Säuren (C₁₀-C₂₀)Fettsäuren sind, die wahlweise an der Kohlenwasserstoffkette hydroxyliert sein können. Unter den an der Kohlenwasserstoffkette hydroxylierten Fettsäuren ist die Ricinolsäure bevorzugt. "Teilweise" verestert bedeutet wie in der Chemie üblich, dass mindestens eine Hydroxyxlgruppe des Zuckeralkohols nicht verestert ist. Der Zuckeralkohol ist bevorzugt Glycerin, Mannit oder Sorbit. Die Anzahl von Oxyethylen-Einheiten im Poly(oxyethylen)derivat kann bevorzugt im Bereich von 5 bis 50, bevorzugt etwa 20 bis etwa 40 liegen. Der Poly(oxyethylen)rest wird, wie in der Technik üblich, durch Umsetzen des teilweise versterten Zuckerlakohols mit Etyhenoxid erhalten. Besonders bevorzugte Beispiele für Emulgatoren in Form von Poly(oxyethylen)derivaten sind die unter den Handelsnamen Cremophor ® (z.B. Cremophor RH 410) und Tween ® (z.B. Tween 20) vertriebenen Emulgatoren.

Die Mengen der Emulgatoren können bevorzugt im Massenverhältnis ovomucinhaltiges Schleimmittel zu Emulgator im Bereich von etwa 10 _{:} 1 bis etwa 1 _{:} 2 eingesetzt werden, wobei das Mengenverhältnis von dem gewünschten Grad der Dispergierung des Ovomucins und/oder von dem allfälligen gleichzeitigen Vorhandensein von schlecht wasserlöslichen Aromastoffen bestimmt sein kann.

Die Gehalte an neutraler Kohlenhydrate können im erfindungsgemäss einzusetzenden Ovomucin enthaltenden Schleimmittel, sofern gewünscht, nach vorbekannten Verfahren bestimmt werden. Die Kohlenhydrate können nach saurer Hydrolyse (TAPPI-Verfahren T 249 cm-85) und Bestimmung der entsprechenden Alditolacetate mittels Flüssig-/Gaschromatographie bestimmt werden *(Teunissen, W., Gosse-link, R.J.A., Vezinhet, F.,* Erfindungsgemäss einsetzbares Ovomucin kann typisch 2 bis 40 Gewichtsprozente neutrale Kohlenhydrate, bevorzugt etwa 20 bis 35 Gewichtsprozente Kohlenhydrate enthalten. Die Gehalte an Kohlenhydrate sind dabei ungefähr gleich wie die entsprechenden durchschnittlichen Gehalte von etwa 33% in Ovomucin laut Literatur (Robinson, D.S., Monsey, J.B., The Biochemical Journal 1966, 100/2, 61-62).

Der Gesamtgehalt an Stickstoff im erfindungsgemäss einzusetzenden Ovomucin enthaltenden Schleimmittel kann wie üblich nach Kjeldahl bestimmt werden. Es kann typisch etwa 11 bis 14 Gewichtsprozente Stickstoff, bevorzugt etwa 12 bis 14 Gewichtsprozent Stickstoff enthalten. Der Gesamtgehalt an Stickstoff kann mittels eines empirischen Faktors von 6,25 in einen Gesamtgehalt an Proteinen umgerechnet werden.

Bevorzugt weisen die erfindungsgemässen Mundpflegemittel in Form von Speichelersatzflüssigkeiten eine dem menschlichen Speichel ähnliche Thixotropie auf. Der Begriff der Thixotropie hat hier die übliche Bedeutung, d.h. er bezeichnet die Abnahme der dynamischen Viskosität η (in Pa s) mit zunehmender Schergeschwindigkeit D (in s⁻¹) bei gegebener, konstanter Temperatur.

Der Begriff "dem menschlichen Speichel ähnliche Thixotropie" kann im Rahmen der vorliegenden Anmeldung bedeuten, dass der Quotient aus der dynamischen Viskosität η einer erfindungsgemässen Speichelersatzflüssigkeit und der dynamischen Viskosität η von menschlichem Speichel für jeden Wert der Schergeschwindigkeit D im Bereich von 60 bis 300 s⁻¹ immer zwischen etwa 0,1 und etwa 10, bevorzugt immer zwischen etwa 0,3 und etwa 3 liegt, wobei alle Messungen bei 37°C vorgenommen werden. Der angegebene Bereich der Schergeschwindigkeit ist typisch für die Scherwirkungen, die innerhalb der Speichelschicht der Mundschleimhäute beim Sprechen und Kauen auftreten.

Menschlicher Speichel selber weist bei 37°C typisch die in Tabelle 1 aufgeführten rheologischen Eigenschaften auf (Speichel-Mischprobe von 11 menschlichen Probanden, Entnahmezeit 14 Uhr). Die Messbedingungen sind hier dieselben wie im Abschnitt "Messung" von Beispiel 3.

**Tabelle 1**

| Scherbeanspruchung D (s⁻¹) | Viskosität η (Pa s) |
|---|---|
| 46,961 | 0,002818 |
| 54,794 | 0,002787 |
| 63,861 | 0,002733 |
| 74,183 | 0,002597 |
| 85,880 | 0,002552 |
| 98,995 | 0,002467 |
| 113,76 | 0,002421 |
| 130,35 | 0,002340 |
| 148,86 | 0,002292 |
| 169,45 | 0,002249 |
| 192,46 | 0,002210 |
| 218,13 | 0,002199 |
| 246,58 | 0,002157 |
| 277,87 | 0,002090 |
| 312,71 | 0,002078 |
| 350,91 | 0,002081 |
| 393,07 | 0,002070 |

Die Werte in Tabelle 1 können als Referenzkurve für den obigen Begriff "dem menschlichen Speichel ähnliche Thixotropie" dienen.

Da die meisten eingangs erwähnten Zusätze die Viskosität und die Thixotropie einer Speichelersatzflüssigkeit beeinflussen, ist es bevorzugt, wenn zunächst Art und Menge der Zusätze so bestimmt werden, wie sie auch in menschlichem Speichel vorkommen, und erst dann mittels einer Versuchsreihe bestimmt wird, wie der Anteil an Ovomucin enthaltendem Schleimmittel sein muss, um eine dem menschlichen Speichel ähnliche Thixotropie zu erzielen. Der Gehalt an Ovomucin enthaltendem Schleimmittel in den erfindungsgemäss bevorzugten Mundpflegemitteln in Form von Speichelersatzflüssigkeiten kann dann typisch in der Grössenordnung vom etwa 0,01 bis etwa 2 Gewichtsprozenten betragen.

Neben dem Ovomucin enthaltenden Schleimmittel können den erfindungsgemässen Mundpflegemitteln wahlweise weitere Schleimmittel, wie sie bereits vorbekannt sind (z.B. Carboxymethylcellulose, Hydroxyalkylcellulosen, wasserlösliche und quellbare Salze der Polyacrylsäuren, Alginate, Carraghenate, Guar-Gum, hochmolekulares Polyethylenoxid, tierische Mucine), zugegeben werden. Die Mengen dieser zusätzlichen weiteren Schleimmittel können typisch etwa 0,5 bis etwa 5 Gewichtsprozente, bezogen auf das fertige Mundpflegemittel, betragen, wobei der viskositätsändernden Wirkung eines solchen zusätzlichen Schleimmittels Rechnung getragen werden kann.

Es kann beispielsweise im Fall einer erfindungsgemässen Speichelersatzflüssigkeit Ovomucin enthaltendes Schleimittel in Kombination mit einem weiteren Schleimmittel verwendet werden, um die Thixotropie möglichst genau an die Thixotropie des menschlichen Speichels anzugleichen. Durch die Überlagerung der rheologischen Eigenschaften des Ovomucins mit den rheologischen Eigenschaften des in Kombination verwendeten zusätzlichen Schleimmittels lassen sich die thixotropen Eigenschaften der erfindungsgemäss bevorzugten Speichelersatzflüssigkeiten unter Umständen feiner abstimmen als nur mit Ovomucin allein. Die zusätzlichen Schleimmittel, die in Kombination mit dem Ovomucin enthaltenden Schleimittel verwendet werden können, können dabei für sich alleine thixotrope oder nicht-thixotrope (newtonsche) Lösungen erzeugen.

Besonders bevorzugt enthalten solche erfindungsgemässen Mundpflegemittel, insbesondere Speichelersatzflüssigkeiten, eine Kombination von Ovomucin enthaltendem Schleimmittel mit Mannoprotein als zusätzlichem Schleimmittel. Mannoprotein ist der generische Oberbegriff für eine Klasse von Polysacchariden, die einen wichtigen Bestandteil der Zellwände sind. Beispiele für Mannoproteine sind die Adhesine in *Candida albicans* und die Agglutinine in *Saccharomyces cerevisiae.* Diese sind als teilgereinigte, getrocknete Präparate teilweise im Handel erhältlich und können in der Form direkt erfindungsgemäss verwendet werden. Bevorzugt sind die Mannoproteine aus *Saccharomyces cerevisae.* Hinsichtlich der Gewinnung und der Eigenschaften von Mannoprotein aus *Saccharomyces cerevisiae* kann auch auf die Literatur verwiesen werden (z.B. Barriga, J.A.T., Cooper, D.G., Idziak, E.S, Cameron, D.R., Enzyme and Microbial Technology 1999, 25, 96-102).

Das Gewichtsverhältnis von Ovomucin enthaltendem Schleimmittel zu Mannoprotein kann in diesen erfindungsgemässen Mundpflegemitteln etwa 1 _{:} 1000 bis etwa 10 _{:} 1 betragen, und vorzugsweise beträgt es 1 _{:} 100 bis etwa 2 : 1. Ovomucin enthaltendes Schleimmittel und Mannoprotein werden vorzugsweise in solchen erfindungsgemässen Mundpflegemitteln, insbesondere in erfindungsgemässen Speichelersatzflüssigkeiten, in Mengen dergestalt eingesetzt, dass Gehalte (in Gewichtsprozenten) an Ovomucin enthaltendem Schleimmittel von etwa 0,01 bis etwa 5 %, und an Mannoprotein von etwa 0,5 bis etwa 10 % resultieren. Besonders bevorzugt werden Ovomucin enthaltendes Schleimmittel und Mannoprotein in Mengen eingesetzt, die etwa 0,05 bis etwa 1,0 % Ovomucin enthaltendes Schleimmittel und etwa 2,0 bis etwa bis etwa 0,5 % Mannoprotein ergeben.

Auch bei Mundpflegemitteln mit einer Kombination von Ovomucin enthaltendem Schleimmittel und Mannoprotein können die weiter vorne erwähnten Methoden zur Löslichkeitsverbesserung des Ovomucins, insbesondere auch die dort erwähnten Emulgatoren, eingesetzt werden. Bevorzugt weist auch hier das Ovomucin enthaltende Schleimmittel einen Sialinsäuregehalt von 1,5 bis 2,5 Gewichtsprozenten, bezogen auf die Trockenmasse, auf.

Den erfindungsgemässen Mundpflegemitteln können in allen Fällen bevorzugt auch weitere Feuchthaltemittel als Zusätze zugegeben werden. Solche sind beispielsweise mehrwertige Alkohole wie Glycerin, Propylenglykol, Sorbit, Mannit, Glucosesirup, Polyethylenglykole oder Polypropylenglykole. Sofern sie verwendet werden, kann ihre Menge typisch etwa 0,5 bis etwa 5 Gewichtsprozente, bezogen auf die fertige Speichelersatzflüssigkeit, betragen.

Den erfindungsgemässen Mundpflegemittel können bevorzugt als Zusätze auch Geschmacksstoffe wie beispielsweise Saccharin, quaternäre Ammoniumsaccharinate, Cyclamate, Cumarin, Vanillin, und Aromastoffe wie etwa Pfefferminzöl, Spearmintöl, Anisöl, Menthol, Anethol, Citrusöl, usw., oder sonstige Aromen wie Apfel-, Eukalyptus- oder Krauseminzaroma zugegeben werden. Sie können typisch in Mengen von etwa 0,5 bis etwa 2 Gewichtsprozenten, bezogen auf das fertige Mundpflegemittel, beigegeben werden, wobei der Stärke und Art des Geschmacks Rechnung getragen werden kann.

Die erfindungsgemässen Mundpflegemittel können auch wahlweise physiologisch verträgliche Konservierungsstoffe wie etwa Natriumbenzoat oder Sorbinsäure in antimikrobiell wirksamen Mengen als Zusätze enthalten.

Sofern eine verstärkte bakterizide oder kariesverhütende Wirkung gewünscht wird, kann den erfindungsgemässen Mundpflegemitteln eine oder mehrere Quellen von Fluoridionen zugesetzt werden. Beispiele hierfür sind wasserlösliche anorganische Fluoridsalze wie NaF, KF und SnF₂, und organische Ammoniumfluoride wie Olaflur (N'-Octadecyl-N',N,N-tris(hydroxyethyl)-1,3-propandiamin-dihydrofluorid) oder die in der internationalen Patentveröffentlichung WO-A-98/22427 beschriebenen Bis-(2-hydroxyethyl)alkylammoni-umfluoride. Diese Fluoridquellen können einzeln oder in Kombination eingesetzt werden. Die Mengen an Fluoridquelle können typisch so gewählt werden, dass das fertige Mundpflegemittel etwa 100 bis etwa 1500 ppm freies Fluorid aufweist.

Die erfindungsgemässen Mundpflegemittel können bevorzugt unter Mischen eines Ovomucin enthaltenden Schleimmittels, insbesondere eines solchen Schleimmittels mit einem Sialinsäuregehalt von 1,5 bis 2,5 Gewichtsprozenten, mit einer wässerigen Lösung, umfassend einen Emulgator, hergestellt werden. Ein besonders bevorzugtes Verfahren zur Herstellung der erfindungsgemässen Mundpflegemittel insbesondere in Form von Speichelersatzflüssigkeiten, Spüllösungen oder Touchierlösungen, umfasst die folgenden Schritte: a) Das aus Eiklar oder Hagelschnüren isolierte, einen Sialinsäuregehalt von etwa 1,5 bis etwa 2,5 Gewichtsprozenten der Trockenmasse aufweisende Schleimmittel wird in einer wässerigen Lösung, die den Emulgator und die allfälligen, für das Mundpflegemittel nach Art und Menge typischen Salze aufweist, suspendiert, b) die Suspension wird unter hohen Scherkräften, bevorzugt mittels eines Rührers nach dem Stator-Rotor-Prinzip (etwa einem Ultraturrax ®), gewünschtenfalls unter Kühlung, gerührt (z.B. mehrmals kurzzeitig, etwa 3 bis 10 mal während je 5 bis 10 Sekunden, mit Ruhepausen von 10 bis 20 Sekunden zwischen zwei Rührgängen), c) die Suspension wird gewünschtenfalls mit Ultraschall behandelt (z.B. während mehrerer Minuten, etwa 1 bis 10 Minuten, unter Verwendung etwa eines Ultraschallbads oder einer Ultraschallsonde, gewünschtenfalls unter Kühlung), d) falls nicht gelöste Reste des Ovomucin enthaltenden Schleimmittels verblieben sind, kann das Mundpflegemittel bevorzugt bis zur Lösungsgleichgewichtseinstellung gerührt werden (z.B. 1 bis 5 Stunden bei etwa 20 bis 30°C, bevorzugt bei etwa Raumtemperatur). Die dann noch verbliebenen ungelöste Reste des Schleimmittels können gewünschtenfalls abfiltriert werden.

Die erfindungsgemässen Mundpflegemittel können in Analogie zu den vorbekannten Speichelersatzflüssigkeiten und je nach der Art des Mundpflegemittels mit den üblichen Applikatoren wie etwa gewöhnliche Flaschen, Pinsel, Quetschflaschen oder wahlweise, unter Verwendung eines geeigneten Treibgases wie etwa CO₂, als Sprühlösungen eingesetzt werden. In letzterem Fall können als Applikatoren Sprayfläschchen dienen.

Die erfindungsgemässen Mundpflegemittel können gewünschtenfalls erst unmittelbar vor Gebrauch aus einer festen Formulierung, die das Ovomucin enthaltende Schleimmittel, die wahlweisen weiteren Zusätze und allfälligen weiteren Schleimmittel enthält, und die beispielsweise in Form eines Pulvers, einer Tablette oder einer Pastille vorliegt, durch Auflösen und Verdünnen in einer geeigneten Menge Wasser und allfälliger weiterer Lösungsmittel wie etwa Alkohol hergestellt werden. Dies kann dann wünschenswert sein, wenn das fertige Mundpflegemittel nicht ausreichend lange haltbar ist. Solche Trockenpräparate zur Herstellung eines erfindungsgemässen Mundpflegemittels sind ein weiterer Gegenstand der Erfindung.

Die Erfindung wird nun durch die folgenden Beispiele weiter veranschaulicht. Diese dienen nur zur Illustration, nicht aber zur Auslegung des Schutzumfangs.

### Beispiel 1: Isolierung von Ovomucin enthaltendem Schleimmittel aus Hühner-Eiklar

Frisch entnommenes Eiklar aus Hühnereiern wurde in dem dreifachen Volumen destillierten Wassers gelöst und der pH der Lösung mit Zitronensäure auf 6 eingestellt. Die erhaltene Lösung wurde während 20 Stunden bei 4°C gerührt. Dann wurde die Lösung während 30 min bei 4°C unter 5500 g zentrifugiert und der Zentrifugationsrückstand zweimal mit destilliertem Wasser, zweimal mit 2 Gew% KCl in Wasser und zweimal mit destilliertem Wasser gewaschen, wobei nach jeder Waschlösung 30 min bei 4°C unter 5500 g zentrifugiert wurde.

Der Gehalt an Trockenmasse dieses Präparates wurde durch Trocknen einer abgewogenen Probe unter Normaldruck während 16 Stunden bei 75°C und anschliessend während 3 Stunden bei 105°C bestimmt. Für die weiteren Versuche wurde das Ovomucin in nicht getrockneter Form, unter Annahme des so bestimmten Trockenmassen-Gehaltes, verwendet. Für eine längere Aufbewahrung des Präparates wurde dieses bei -20°C gelagert.

### Beispiel 2: Chromatographische Trennung von Ovomucin enthaltendem Schleimmittel

Die Trennung wurde in Anlehnung an bekannte Verfahren durchgeführt (Awadé, A.C., Moreau, S., Mollé, D., Brulé, G., Maubois, J.L., J. Chrom. A. 1994, 677, 279-288). Es wurde eine Lösung durch Lösen einer Menge Ovomucin enthaltendem Schleimmittel aus Herstellungsbeispiel 1, die 5 mg Trockenmasse entsprach, in 10 ml Wasser, hergestellt und diese Lösung 20 Stunden bei 20°C stehengelassen. 500 µl der Lösung wurden mittels Gelpermeationschromatographie (Trennung nach Molekulargewicht) auf einer Superose 6-Säule (Amersham Pharmacia Biotech, trennbarer MG-Bereich 5 bis 5000 kDa) aufgetrennt. Die mobile Phase war 50 mM Imidazol, 0,2 Volumenprozent 2-Mercaptoethanol, 0,5 Gewichtsprozent Na-Dodecylsulfat (SDS) in Wasser bei pH 7, die Elutionsgeschwindigkeit betrug 0,45 ml/min. Die eluierten Fraktionen wurden mittels Messung der UV-Absorption bei 280 nm detektiert. Es wurde ein Chromatogramm wie in Figur 1 gezeigt erhalten. Da das im Schleimmittel enthaltene Ovomucin im Totvolumen eluiert wird (Pike 1) ist anzunehmen, dass es ein Molekulargewicht von mindestens 5000 kDa aufweist.

### Beispiel 3: Messung rheologischer Eigenschaften von Lösungen mit Ovomucin enthaltendem Schleimmittel

### Probenvorbereitung

Eine Probe des Ovomucin enthaltenden Schleimmittels von Beispiel 1 wurde in PBS-Puffer (0,2 g/l KH₂PO₄, 1,441 g/l Na₂HPO₄ x 2 H₂O, 8,5 g/l NaCl, 0, 1 g/ MgCl₂ x 6 H₂O, 0, 2 g/l KCl; pH 7) mittels eines Ultraturrax und unter sanftem Rühren gelöst. Die Einwage der Ovomucin-Probe war dergestalt, dass eine Lösung von 2 Gewichtsprozenten (bezogen auf die Trockenmasse des Schleimmittels) resultierte. Die fertige Lösung wurde vor den Messungen über Nacht bei 4°C gelagert.

### Messung

Die rheologischen Messungen wurden auf einem dynamischen Stress-Rheometer SR-200 (Rheometric Scientific Inc., Piscataway, USA) bei 37°C durchgeführt. Es wurde eine chrombeschichtete Couette-Zelle von 16 ml Fassungsvermögen verwendet. Vor der eigentlichen Messung wurde die Probe einer Vorbehandlung mit einer Scher-Beanspruchung von ungefähr 100 s⁻¹ während einer Minute und einer anschliessenden Erholungsphase von 3 min unterzogen.

Zur Messung der Viskosität in Abhängigkeit von der Scherbeanspruchung (Figuren 1 und 2) wurde bei den geringstmöglichen Beanspruchungen, die das Messgerät erzeugen konnte, begonnen und die mechanische Einwirkung bis auf 50 Pa erhöht. Es wurde die Scherbeanspruchung und die Viskosität gemessen.

Zur Messung der Wiederherstellung der Viskosität (Figur 3) wurde die Probe der höchstmöglichen Scherbeanspruchung, die das Messgerät erzeugen konnte, unterzogen und anschliessend der zeitliche Verlauf der Viskosität bei der niedrigstmöglichen Scherbeanspruchung, die das Messgerät erzeugen konnte, aufgezeichnet.

### Beispiel 4: Formulierungen für erfindungsgemässe Mundpflegemittel

Die allgemeine Herstellungsvorschrift für die nachfolgenden Beispiele 4a bis 4j war wie folgt:

Die Mundpflegemittel dieser Beispiele (je 100 g) wurden aus bis zu 6 verschiedenen wässerigen Lösung A, B, C, D, E und F hergestellt.

Lösung A (sofern eingesetzt) enthielt die Konservierungsstoffe. Zur Herstellung der Lösung A wurden die Konservierungsstoffe in denjenigen Mengen, wie in der Tabelle des jeweiligen Beispiels angegeben, eingewogen und bei 50°C in 10 g gereinigtem Wasser gelöst.

Lösung B wurde durch Lösen der in der jeweiligen Tabelle angegebenen Menge des Ovomucin enthaltenden Schleimmittels, wie in Beispiel 1 hergestellt, in 20 g gereinigtem Wasser bei maximal 30°C hergestellt.

Lösung C (sofern eingesetzt) enthielt allfällige zusätzliche Feuchthalte- und Schleimmittel (neben Ovomucin enthaltendem Schleimmittel). Hierzu wurden die entsprechenden Mengen dieser Mittel, wie in der jeweiligen Tabelle angegeben, eingewogen und in 15 g gereinigtem Wasser unter Erwärmen auf maximal 30°C gelöst.

Lösung D enthielt die Aromastoffe (sofern eingesetzt) und die möglicherweise erforderlichen Emulgatoren. Hierzu wurden zunächst die Emulgatoren in denjenigen Mengen, wie in der jeweiligen Tabelle angegeben, eingewogen und in 10 g Wasser gelöst. Anschliessend wurde die eingewogene Menge Aromastoffe, wie in der jeweiligen Tabelle angegeben, zugesetzt und es wurde unter Rühren gelöst.

Lösung E enthielt weitere Zusätze, wie etwa Puffersalze, Süssstoffe (künstliche und/oder natürliche), Farbstoffe, Agentien zur Regulierung zur des osmotischen Drucks und der Tonizität. Hierzu wurden die Zusätze, wie in der jeweiligen Tabelle angegeben, in den entsprechenden Mengen eingewogen und in 10 g Wasser gelöst.

Lösung F war eine Stammlösung der wahlweisen Fluoridionengeber NaF oder Olaflur in Wasser. Im Fall von NaF betrug die Konzentration Stammlösung 50 mg NaF / 100 ml Lösung und im Fall von Olaflur betrug sie 500 mg / 100 ml Lösung. Bei der Herstellung der Mundpflegemittel wurde, sofern eine Fluoridionenquelle gewünscht war, ein Aliquot NaF- oder Olaflur-Stammlösung mitverwendet.

Zur Herstellung des fertigen Mundpflegemittels wurde zunächst Lösung B vorgelegt. Unter Rühren bei Raumtemperatur wurde Lösung A (sofern eingesetzt), dann Lösung C (sofern eingesetzt), dann Lösung D (sofern eingesetzt), dann Lösung E, dann das restliche, für 100 g Mundpflegemittel benötigte Wasser, soweit es nicht bereits zur Herstellung der übrigen, zu verwendenden Ausgangslösungen verwendet worden war, und am Schluss, sofern verwendet, das oben erwähnte Aliquot von Stammlösung F zugegeben. Nach Zugabe jeder der Lösungen wurde bis zur völligen Homogenisierung gerührt.

### Beispiel 4a (Speichelersatzflüssigkeit):

| | Gramm | Lösung |
|---|---|---|
| Ovomucin enthaltendes Schleimmittel | 0,100 | B |
| Hydroxyethylcellulose | 2,000 | C |
| Methylparaben | 0,180 | A |
| Propylparaben | 0,020 | A |
| Aroma | 0,800 | D |
| Cremophor ® | 0,200 | D |
| K₂HP0₄ | 0,035 | E |
| KHC0₃ | 0,150 | E |
| KCl | 0,122 | E |
| NaCl | 0,086 | E |
| MgCl₂ | 0,005 | E |
| CaCl₂ | 0,015 | E |
| Sorbitol | 3,000 | E |
| Na-Saccharin | 0,100 | E |
| Wasser | ad 100 | |

### Beispiel 4b (Speichelersatzflüssigkeit):

| | Gramm | Lösung |
|---|---|---|
| Ovomucin enthaltendes Schleimmittel | 0,100 | B |
| Mannoprotein | 3,000 | C |
| Methylparaben | 0,180 | A |
| Propylparaben | 0,020 | A |
| Aroma | 0,800 | D |
| Cremophor ® | 0,200 | D |
| K₂HPO₄ | 0,035 | E |
| KHCO₃ | 0,150 | E |
| KCl | 0, 122 | E |
| NaCl | 0,086 | E |
| MgCl₂ | 0,005 | E |
| CaCl₂ | 0,015 | E |
| Na-Saccharin | 0,100 | E |
| Wasser | ad 100 | |

### Beispiel 4c (Speichelersatzflüssigkeit):

| | Gramm | Lösung |
|---|---|---|
| Ovomucin enthaltendes Schleimmittel | 0,500 | B |
| Carboxymethylcellulose | 1,500 | C |
| Natriumbenzoat | 0,200 | A |
| Sorbinsäure | 0,100 | A |
| Tween ® 20 | 0,15 | D |
| K₂HPO₄ | 0,035 | E |
| KHCO₃ | 0,150 | E |
| KCl | 0,122 | E |
| NaCl | 0,086 | E |
| MgCl₂ | 0,005 | E |
| CaCl₂ | 0,015 | E |
| Xylitol | 2,000 | E |
| Saccharin | 0,200 | E |
| NaF | 0,0005 | F (Aliquot von 1 ml |
| Wasser | ad 100 | |

### Beispiel 4d (Speichelersatzflüssigkeit):

| | Gramm | Lösung |
|---|---|---|
| Ovomucin enthaltendes Schleimmittel | 0,500 | B |
| Mannoprotein | 5,000 | C |
| Methylparaben | 0,180 | A |
| Propylparaben | 0,020 | A |
| Aroma | 0,800 | D |
| Cremophor ® | 0,200 | D |
| K₂HPO₄ | 0,035 | E |
| KHC0₃ | 0, 150 | E |
| KCl | 0,122 | E |
| NaCl | 0,086 | E |
| M₉Cl₂ | 0,005 | E |
| CaCl₂ | 0,015 | E |
| Saccharin | 0,200 | E |
| Wasser | ad 100 | |

### Beispiel 4e (Speichelersatzflüssigkeit):

| | Gramm | Lösung |
|---|---|---|
| Ovomucin enthaltendes Schleimmittel | 0,750 | B |
| Hydroxyethylcellulose | 1,000 | C |
| Aroma | 0,700 | D |
| Tween ® 20 | 0,2 | D |
| Na₂HP0₄ | 0,028 | E |
| KHC0₃ | 0,150 | E |
| KCl | 0, 120 | E |
| NaCl | 0,005 | E |
| MgCl₂ | 0,005 | E |
| CaCl₂ | 0,015 | E |
| Mannitol | 2,000 | E |
| Na-Saccharin | 0,200 | E |
| Wasser | ad 100 | |

### Beispiel 4f (Speichelersatzflüssigkeit)

| | Gramm | Lösung |
|---|---|---|
| Ovomucin enthaltendes Schleimmittel | 0,750 | B |
| Mannoprotein | 2,000 | C |
| Natriumbenzoat | 0,200 | A |
| Sorbinsäure | 0,100 | A |
| Aroma | 0,700 | D |
| Cremophor ® | 0,200 | D |
| Na₂HP0₄ | 0,028 | E |
| KHCO₃ | 0,150 | E |
| KCl | 0,120 | E |
| NaCl | 0,005 | E |
| MgCl₂ | 0,005 | E |
| CaCl₂ | 0,015 | E |
| Na-Saccharin | 0,200 | E |
| Wasser | ad 100 | |

### Beispiel 4g (Speichelersatzflüssigkeit):

| | Gramm | Lösung |
|---|---|---|
| Ovomucin enthaltendes Schleimmittel | 1,000 | B |
| Carboxymethylcellulose | 1,000 | C |
| K₂HPO₄ | 0,035 | E |
| KCl | 0,122 | E |
| KHCO₃ | 0,150 | E |
| NaCl | 0,086 | E |
| MgCl₂ | 0,005 | E |
| CaCl₂ | 0,015 | E |
| Xylitol | 3,000 | E |
| Saccharin | 0,200 | E |
| Aroma | 0,900 | D |
| Cremophor ® | 0,200 | D |
| Natriumbenzoat | 0,200 | A |
| Sorbinsäure | 0,100 | A |
| Wasser | ad 100 | |

### Beispiel 4h (Speichelersatzflüssigkeit):

| | Gramm | Lösung |
|---|---|---|
| Ovomucin enthaltendes Schleimmittel | 1,000 | B |
| Mannoprotein | 2,000 | C |
| Methylparaben | 0,180 | A |
| Propylparaben | 0,020 | A |
| Aroma | 0,900 | D |
| K₂HPO₄ | 0,035 | E |
| KHC0₃ | 0,150 | E |
| KCl | 0,122 | E |
| NaCl | 0,086 | E |
| MgCl₂ | 0,005 | E |
| CaCl₂ | 0,015 | E |
| Saccharin | 0,200 | E |
| Olaflur | 0,0035 | F (Aliquot von 7 ml) |
| Wasser | ad 100 | |

### Beispiel 4i (Spüllösung):

| | Gramm | Lösung |
|---|---|---|
| Ovomucin enthaltendes Schleimmittel | 0,100 | B |
| Xylit | 2,500 | C |
| Aroma (Pfefferminz/Krauseminz) | 0,100 | D |
| PEG-40-hydriertes Rizinusöl (Cremophor ® RH 410, BASF) | 0,200 | D |
| 0,4%ige Pigmentlösung von Ariavit Blau 3.85 CI 42051 | 0,050 | E |
| Acesulfam K | 0,025 | E |
| Ethanol | 5,000 | E |
| Olaflur | 0,125 | F (Aliquot von 25 ml) |
| Wasser | ad 100 | |

### Beispiel 4j (Spüllösung):

| | Gramm | Lösung |
|---|---|---|
| Ovomucin enthaltendes Schleimmittel | 0,100 | B |
| Mannoprotein | 2,500 | C |
| Aroma (Pfefferminz/Krauseminz) | 0, 100 | D |
| PEG-40-hydriertes Rizinusöl (Cremophor ® RH 410, BASF) | 0,200 | D |
| 0,4%ige Pigmentlösung von Ariavit Blau 3.85 CI 42051 | 0,050 | E |
| Acesulfam K | 0,025 | E |
| Ethanol | 5,000 | E |
| Olaflur | 0,125 | F (Aliquot von 25 ml) |
| Wasser | ad 100 | |

### Beispiel 4k (Touchierlösung):

| | Gramm | Lösung |
|---|---|---|
| Ovomucin enthaltendes Schleimmittel | 0,100 | B |
| Aroma (bestehend aus 30 Teilen Anisöl, 7,5 Teilen Menthol, 1,0 Teilen Vanillin, 6,0 Teilen Krauseminzöl und 55,5 Teilen Pfefferminzöl) | 2,500 | D |
| Tween ® 20 | 0,2 | D |
| Olaflur | 10,000 | ungelöst, fest eingewogen. |
| Saccharin | 0,150 | E |
| Wasser | ad 100 | |

Es wurde im Beispiel 4k derselbe Verfahrensbeschrieb wie für die Beispiele 4a bis 4j befolgt, ausser dass zunächst 10,000 g Olaflur eingewogen wurden und anschliessend die Lösungen B, D, E und das restliche Wasser, wie im allgemeinen Verfahrensbeschrieb angegeben, zugegeben wurden.

### Beispiel 41 (Speichelersatzflüssigkeit):

| | Gramm | Lösung |
|---|---|---|
| Ovomucin enthaltendes Schleimmittel | 0,100 | B |
| Hydroxyethylcellulose | 2,000 | C |
| Methylparaben | 0,180 | A |
| Propylparaben | 0,020 | A |
| Aroma | 0,800 | D |
| Tween ® 20 | 0,200 | D |
| K₂HPO₄ | 0,035 | E |
| KHC0₃ | 0, 150 | E |
| KCl | 0,122 | E |
| NaCl | 0,086 | E |
| MgCl₂ | 0,005 | E |
| CaCl₂ | 0,015 | E |
| Sorbitol | 3,000 | E |
| Na-Saccharin | 0,100 | E |
| Wasser | ad 100 | |

### Beispiel 4m (Speichelersatzflüssigkeit):

| | Gramm | Lösung |
|---|---|---|
| Ovomucin enthaltendes Schleimmittel | 0,100 | B |
| Mannoprotein | 3,000 | C |
| Methylparaben | 0,180 | A |
| Propylparaben | 0,020 | A |
| K₂HP0₄ | 0,035 | E |
| KHC0₃ | 0,150 | E |
| KCl | 0,122 | E |
| NaCl | 0,086 | E |
| MgCl₂ | 0,005 | E |
| CaCl₂ | 0,015 | E |
| Na-Saccharin | 0,100 | E |
| Wasser | ad 100 | |

### Beispiel 4n (Speichelersatzflüssigkeit):

| | Gramm | Lösung |
|---|---|---|
| Ovomucin enthaltendes Schleimmittel | 0,500 | B |
| Mannoprotein | 5,000 | C |
| Methylparaben | 0,180 | A |
| Propylparaben | 0,020 | A |
| Aroma | 0,800 | D |
| Tween ® 20 | 0,200 | D |
| K₂HPO₄ | 0,035 | E |
| KHC0₃ | 0,150 | E |
| KCl | 0,122 | E |
| NaCl | 0,086 | E |
| MgCl₂ | 0,005 | E |
| CaCl₂ | 0,015 | E |
| Saccharin | 0,200 | E |
| Wasser | ad 100 | |

### Beispiel 4o (Speichelersatzflüssigkeit)

| | Gramm | Lösung |
|---|---|---|
| Ovomucin enthaltendes Schleimmittel | 0,750 | B |
| Mannoprotein | 2,000 | C |
| Natriumbenzoat | 0,200 | A |
| Sorbinsäure | 0,100 | A |
| Aroma | 0,700 | D |
| Tween ® 20 | 0,200 | D |
| Na₂HP0₄ | 0,028 | E |
| KHC0₃ | 0,150 | E |
| KCl | 0,120 | E |
| NaCl | 0,005 | E |
| MgCl₂ | 0,005 | E |
| CaCl₂ | 0,015 | E |
| Na-Saccharin | 0,200 | E |
| Wasser | ad 100 | |

### Beispiel 4p (Speichelersatzflüssigkeit):

| | Gramm | Lösung |
|---|---|---|
| Ovomucin enthaltendes Schleimmittel | 1,000 | B |
| Carboxymethylcellulose | 1,000 | C |
| K₂HPO₄ | 0,035 | E |
| KCl | 0,122 | E |
| KHCO₃ | 0, 150 | E |
| NaCl | 0,086 | E |
| MgCl₂ | 0,005 | E |
| CaCl₂ | 0,015 | E |
| Xylitol | 3,000 | E |
| Saccharin | 0,200 | E |
| Aroma | 0,900 | D |
| Tween ® 20 | 0,200 | D |
| Natriumbenzoat | 0,200 | A |
| Sorbinsäure | 0,100 | A |
| Wasser | ad 100 | |

### Beispiel 4q (Spüllösung):

| | Gramm | Lösung |
|---|---|---|
| Ovomucin enthaltendes Schleimmittel | 0,100 | B |
| Xylit | 2,500 | C |
| Aroma (Pfefferminz/Krauseminz) | 0,100 | D |
| Tween ® 20 | 0,200 | D |
| 0,4%ige Pigmentlösung von Ariavit Blau 3.85 CI 42051 | 0,050 | E |
| Acesulfam K | 0,025 | E |
| Ethanol | 5,000 | E |
| Olaflur | 0,125 | F (Aliquot von 25 ml) |
| Wasser | ad 100 | |

### Beispiel 4r (Spüllösung):

| | Gramm | Lösung |
|---|---|---|
| Ovomucin enthaltendes Schleimmittel | 0,100 | B |
| Mannoprotein | 2,500 | C |
| Aroma (Pfefferminz/Krauseminz) | 0,100 | D |
| Tween ® 20 | 0,200 | D |
| 0,4%ige Pigmentlösung von Ariavit Blau 3.85 CI 42051 | 0,050 | E |
| Acesulfam K | 0,025 | E |
| Ethanol | 5,000 | E |
| Olaflur | 0,125 | F (Aliquot von 25 ml) |
| Wasser | ad 100 | |

### Beispiel 5: Messung rheologischer Eigenschaften in einer erfindungsgemässen Speichelersatzflüssigkeit

### Herstellung der Speichelersatzflüssigkeit

Eine Probe eines Ovomucin enthaltenden Schleimmittels (wie in Beispiel 1 hergestellt), die 4 g Trockenmasse enthielt, wurde in 200 ml PBS-Puffer (1,102 g/l KH₂PO₄, 0,262 g/l Na₂HPO₄ x 2 H₂O 0,585 g/l NaCl, 0,1 g/ MgCl₂ x 6 H₂O, 0,2 g/l KCl; pH 6,5) suspendiert. Die Suspension wurde zunächst mit einem Rührer nach dem Stator-Rotor-Prinzip (Ultraturrax IKA T25 basic, Stabdurchmesser 1,8 cm) 3 mal je 20 Sekunden bei 11500 U/min unter Kühlung mit einem Eisbad gerührt, wobei zwischen zwei Rührgängen 15 Sekunden Ruhepause lagen. Danach wurde die Suspension mittels einer Ultraschallsonde (Branson Sonifier 450, Stabdurchmesser 1,2 cm) 20 mal während je 5 Sekunden mit Stufe 6 und unter Kühlung in einem Eisbad mit Ultraschall beschallt, wobei zwischen jeder Beschallung 15 Sekunden Ruhepause waren. Dann wurden 0,3 g Emulgator (Cremophor ® RH 410 bzw. Tween ® 20) zugegeben und die fertige Speichelersatzflüssigkeit während 2 Stunden bei Raumtemperatur gerührt. Zur Messung wurde nur der Überstand der Lösung verwendet; gemäss Sialinsäure-Gehaltsbestimmung waren mindestens etwa 80 Gewichsprozente des eingesetzten Ovomucin enthaltenden Schleimmittels gelöst.

### Messung

Die rheologischen Messungen wurden auf einem dynamischen Stress-Rheometer SR-200 (Rheometric Scientific Inc., Piscataway, USA) bei 37°C durchgeführt. Es wurde eine 40 mm-Zelle mit chrombeschichteten parallelen Platten mit einem Abstand von 0,200 mm verwendet. Vor der eigentlichen Messung wurde die Probe der Speichelersatzflüssigkeit einer Vorbehandlung mit einer Scher-Beanspruchung von ungefähr 100 s⁻¹ während einer Minute und einer anschliessenden Erholungsphase von 3 min unterzogen. Zur eigentlichen Messung wurde bei den geringstmöglichen Beanspruchungen, die das Messgerät erzeugen konnte, begonnen und die Beanspruchung bis auf 50 Pa erhöht.

Es wurde die Scherbeanspruchung und die Viskosität von drei so hergestellten und vorbehandelten Proben der Speichelersatzflüssigkeit gemessen, und das Verhalten wie in Figuren 4 bzw. 5 gezeigt erhalten.

## Patentansprüche

1. Mundpflegemittel umfassend
a) ein Schleimmittel, das Ovomucin enthält, und
b) Mannoprotein.

2. Mundpflegemittel nach Anspruch 1, **dadurch gekennzeichnet, dass** das Gewichtsverhältnis Schleimmittel zu Mannoprotein 1 _{:} 1000 bis 10 _{:} 1 und vorzugsweise 1 . 100 bis 2 _{:} 1 beträgt.

3. Mundpflegemittel nach Anspruche 1 oder 2, umfassend einen Emulgator.

4. Mundpflegemittel nach Anspruch 3, **dadurch gekennzeichnet, dass** der Emulgator ein synthetischer Emulgator ist.

5. Mundpflegemittel nach Anspruch 4, **dadurch gekennzeichnet, dass** der Emulgator ein Poly(oxyethylen)derivat eines teilweise veresterten (C₃-C₆)Zuckeralkohols ist, wobei die den Zuckeralkohol veresternden Säuren (C₁₀-C₂₀)Fettsäuren sind, die wahlweise an der Kohlenwasserstoffkette hydroxyliert sind.

6. Mundpflegemittel nach Anspruch 5, **dadurch gekennzeichnet, dass** das Poly(oxyethylen)derivat 5 bis 50 Oxyethyleneinheiten, bevorzugt 20 bis 40 Oxyethyleneinheiten, umfasst.

7. Mundpflegemittel nach Anspruch 4, **dadurch gekennzeichnet, dass** der Emulgator zwitterionisch ist.

8. Mundpflegemittel nach einem der Ansprüche 1 bis 7, **dadurch gekennzeichnet, dass** es eine Speichelersatzflüssigkeit ist.

9. Speichelersatzflüssigkeit nach Anspruch 8, **dadurch gekennzeichnet, dass** sie Zusätze enthält, die aus NaCl, CaCl₂, MgCl₂, KCl, den Dihydrogenphosphaten und Hydrogenphosphaten des Natriums oder Kaliums, und den Carbonaten und Hydrogencarbonaten des Natriums oder Kaliums ausgewählt sind.

10. Speichelersatzflüssigkeit nach Anspruch 9, **dadurch gekennzeichnet, dass** die Zusätze 0,05 bis 0,15 Gewichtsprozente KCl; 0,05 bis 0,1 Gewichtsprozente NaCl; 0,002 bis 0,008 Gewichtsprozente MgCl₂ und 0,01 bis 0,02 Gewichtsprozente CaCl₂ sind und dass sie mit Dihydrogenphosphat/Hydrogenphosphat und/oder Carbonat-/Hydrogencarbonat-Puffer auf einen pH-Wert und eine Pufferkapazität gepuffert ist, die dem menschlichen Speichel entspricht.

11. Speichelersatzflüssigkeit nach Anspruch 10, **dadurch gekennzeichnet, dass** sie etwa 0,12 Gewichtprozente KCl, etwa 0,086 Gewichtsprozente NaCl, etwa 0,005 Gewichtsprozente MgCl₂ und etwa 0,015 Gewichtsprozente CaCl₂ enthält.

12. Speichelersatzflüssigkeit nach einem der Ansprüche 8 bis 11, mit einer dem menschlichen Speichel ähnlichen Thixotropie.

13. Mundpflegemittel nach einem der Ansprüche 1 bis 12, **dadurch gekennzeichnet, dass** es als Zusatz ein Feuchthaltemittel enthält.

14. Mundpflegemittel nach Anspruch 13, **dadurch gekennzeichnet, dass** das Feuchthaltemittel aus Glycerin, Propylenglykol, Sorbit, Mannit, Glucosesirup und den Polyethylenglykolen oder Polypropylenglykolen ausgewählt ist.

15. Feste Formulierung enthaltend eine Kombination aus einem Ovomucin enthaltenden Schleimmittel mit oder mehreren weiteren e n, die aus der Gruppe Mannoprotein und enthaltend wahlweise weitere Zusätze dergestalt, dass die Formulierung beim Auflösen in Wasser oder in einer Mischung aus Wasser und Ethanol ein Mundpflegemittel nach anspruch ergibt.
